# NEW EUROPEAN PATENT SPECIFICATION

(11) **EP 0 428 267 B2**
(45) Date of publication and mention of the opposition decision: **08.12.2004**
(45) Mention of the grant of the patent: 11.12.1996
(21) Application number: 90311193.8
(22) Date of filing: 12.10.1990
(51) Int. Cl.: C07K 14/505, C12N 15/16

(54) **Erythropoietin isoforms**
Erythropoietin-Isoformen
Isoformes d'érythropoiétin

(30) Priority: 13.10.1989 US 421444; 09.10.1990 WO PCT/US90/05758
(43) Date of publication of application: 22.05.1991
(60) Divisional application: 95101849.8
(73) Proprietor: Kirin-Amgen, Inc., 6002 Lucerne (CH)
(72) Inventor: Strickland, Thomas Wayne, Moorpark, CA 93021 (US); Byrne, Thomas Edward, Bethesda, MD 20817 (US); Elliott, Steven George, Thousand Oaks, CA 91320 (US)
(74) Representative: Brown, John David

(56) References cited:
- EP-A- 0 148 605
- EP-A- 0 267 678
- JOURNAL OF BIOLOGICAL CHEMISTRY, vol. 263, no. 8, 15 March 1988, American Society for Biochemistry & Molecular Biology Inc., US; M. TAKEUCHI et al., pp. 3657-3659
- JOURNAL OF BIOCHEMISTRY, vol. 107, no. 3, March 1990, Tokyo (JP); N. IMAI et al., pp. 352-359
- PROCEEDINGS OF THE NATL. ACADEMY OF SCIENCES USA, vol. 82, no. 22, November 1985, Washington, DC (US); F.-K. LIN et al., pp. 7580-7584
- CHEMICAL ABSTRACTS, vol. 102, no. 3, 1984, Columbus, OH (US); D.C. PHELPS et al., & BIOCHIM. BIOPHYS, ACTA., 791("), 226-238
- BIOCHEMICAL & BIOPHYSICAL RESEARCH COMMUNICATIONS, vol. 98, no. 4, 27 February 1981, Academic Press Inc.; J.E. FUHR et al., pp. 930-935

## Description

This is a continuation-in-part application of U.S. application Ser.No. 421,444, filed October 13, 1989, which is incorporated by reference. The present invention relates to erythropoietin isoforms or mixtures thereof, to the methods for the preparation of specific isoforms or mixtures thereof, to pharmaceutical compositions comprising such isoforms or mixtures thereof, and to methods of treatment utilizing such isoforms and compositions.

### Background of the Invention

Erythropoietin is a glycoprotein hormone involved in the maturation of erythroid progenitor cells into erythrocytes. It is essential in regulating levels of red blood cells in circulation. Naturally occurring erythropoietin is produced by the liver during fetal life and by the kidney of adults and circulates in the blood and stimulates the production of red blood cells in bone marrow. Anemia is almost invariably a consequence of renal failure due to decreased production of erythropoietin from the kidney. Recombinant erythropoietin produced by genetic engineering techniques involving the expression of a protein product from a host cell transformed with the gene encoding erythropoietin has been found to be effective when used in the treatment of anemia resulting from chronic renal failure.

Until recently, the availability of erythropoietin has been very limited. Although the protein is present in human urine, excreted levels are too low to make this a practical source of erythropoietin for therapeutic use. Patients suffering from aplastic anemia exhibit elevated levels of urinary erythropoietin relative to healthy individuals, but limited supplies of this urine also make such a source impractical. The purification of human urinary erythropoietin by Miyake et al. in J. Biol. Chem., 252, 5558 (1977), used, as starting material, urine from aplastic anemic individuals.

The identification, cloning, and expression of genes encoding erythropoietin are described in U. S. patent 4,703,008 to Lin. A description of the purification of recombinant erythropoietin from cell medium that supported the growth of mammalian cells containing recombinant erythropoietin plasmids for example, is included in U. S. patent 4,667,016 to Lai et al. The expression and recovery of biologically active recombinant erythropoietin from mammalian cell hosts containing the erythropoietin gene on recombinant plasmids has, for the first time, made available quantities of erythropoietin suitable for therapeutic applications. In addition, knowledge of the gene sequence and the availability of larger quantities of purified protein has led to a better understanding of the mode of action of this protein.

The biological activity of a protein is dependent upon its structure. In particular, the primary structure of a protein (i.e., its amino acid sequence) provides information that allows the formation of secondary (e.g, α helix or β-sheet) and tertiary (overall three-dimensional folding) structures by a polypeptide during and after its synthesis. The disruption of proper secondary and tertiary structures by the introduction of mutations or by chemical or enzymatic treatments can result in a reduction in biological activity.

In procaryotic organisms, the biological activities of proteins are largely governed by the structures described above. Unlike proteins from procaryotic cells, many cells surface and secretory proteins produced by eucaryotic cells are modified with one or more oligosaccharide groups. This modification, referred to as glycosylation, can dramatically affect the physical properties of proteins and can also be important in protein stability, secretion, and subcellular localization. Proper glycosylation can be essential for biological activity. In fact, some genes from eucaryotic organisms, when expressed in bacteria (e.g., E. coli) which lack cellular processes for glycosylating proteins, yield proteins that are recovered with little or no activity by virtue of their lack of glycosylation.

Glycosylation occurs at specific locations along the polypeptide backbone and is usually of two types: O-linked oligosaccharides are attached to serine or threonine residues while N-linked oligosaccharides are attached to asparagine residues when they are part of the sequence Asn-X-Ser/Thr, where X can be any amino acid except proline. The structures of N-linked and O-linked oligosaccharides and the sugar residues found in each type are different. One type of sugar that is commonly found on both is N-acetylneuraminic acid (hereafter referred to as sialic acid). Sialic acid is usually the terminal residue of both N-linked and O-linked oligosaccharides and, by virtue of its negative charge, may confer acidic properties to the glycoprotein.

Both human urinary derived erythropoietin and recombinant erythropoietin (expressed in mammalian cells) having the amino acid sequence 1-165 of human erythropoietin contain three N-linked and one O-linked oligosaccharide chains which together comprise about 40% of the total molecular weight of the glycoprotein. N-linked glycosylation occurs at asparagine residues located at positions 24, 38 and 83 while O-linked glycosylation occurs at a serine residue located at position 126 (Lai et al. J. Biol. Chem. 261, 3116 (1986); Broudy et al. Arch. Biochem. Biophys. 265, 329 (1988)). The oligosaccharide chains have been shown to be modified with terminal sialic acid residues. Enzymatic treatment of glycosylated erythropoietin to remove all sialic acid residues results in a loss of in vivo activity but does not affect in vitro activity (Lowy et al. Nature 185, 102 (1960); Goldwasser et al. J. Biol. Chem. 249, 4202 (1974)). This behavior has been explained by rapid clearance of asialoerythropoietin from circulation upon interaction with the hepatic asialoglycoprotein binding protein (Morrell et al. J. Biol. Chem. 243, 155 (1968); Briggs, et al. Am. J. Physiol. 227, 1385 (1974); Ashwell et al. Methods Enzymol. 50, 287 (1978)). Thus, erythropoietin possesses in vivo biological activity only when it is sialylated to avoid its binding by the hepatic binding protein.

The role of the other components in the oligosaccharide chains of erythropoietin is not well defined. It has been shown that non-glycosylated erythropoietin has greatly reduced in vivo activity compared to the glycosylated form but does retain in vitro activity (Dordal et al. Endocrinology 116, 2293 (1985); Lin patent, supra) . In another study, however, the removal of N-linked or O-linked oligosaccharide chains singly or together by mutagenesis of asparagine or serine residues that are glycosylation sites sharply reduces in vitro activity of the altered erythropoietin that is produced in mammalian cells (Dube be et al. J. Biol. Chem. 263, 17516 (1988)).

Glycoproteins such as erythropoietin can be separated into different charged forms using techniques such as isoelectric focusing (IEF). Several parties have reported IEF studies of crude and partially purified erythropoietin preparations (Lukowsky et al., J. Biochem 50, 909 (1972); Shelton et al. Biochem. Med. 12, 45 (1975); Fuhr et al. Biochem. Biophys. Res. Comm. 98, 930 (1981)). At most, three or four fractions having erythropoietin activity were distinguished by IEF in these studies and none were characterized with respect to carbohydrate content. In addition, no correlation between the isoelectric points of the fractions and their biological activity was made.

During the purification of urinary erythropoietin from human urine discussed in Miyake et. al. supra, two erythropoietin fractions from hydroxylapatite chromatography designated II and IIIA were reported to have the same specific activity. A subsequent carbohydrate analysis of fractions II and IIIA revealed that fraction II had a greater average sialic acid content than fraction IIIA (Dordal et. al. supra).

It is an object of the present invention to provide separated and isolated isoforms of erythropoietin having a defined sialic acid content and biological activity. Pharmaceutical compositions containing such molecules would have therapeutic benefit.

### Summary of the Invention

The subject invention relates to erythropoietin isoforms. Also provided is a method of preparing an erythropoietin isoform comprising the steps of subjecting purified erythropoietin to preparative isoelectric focusing, and eluting a single isoform from the gel. Pharmaceutically acceptable compositions comprising erythropoietin isoforms are also provided. This invention also relates to methods of increasing hematocrit levels in mammals comprising administering a therapeutically acceptable amount of these compositions to increase production of reticulocytes and red blood cells.

The subject invention relates to a method of preparing a mixture of erythropoietin molecules having greater than or alternatively less than a predetermined number of sialic acids per molecule comprising subjecting material containing erythropoietin to ion exchange chromatography. Also comprised by the subject invention is a method of preparing a mixture of erythropoietin molecules having greater than or alternatively less than a predetermined number of sialic acids per molecule comprising subjecting a material containing erythropoietin to chromatofocusing.

### Brief Description of the Drawings

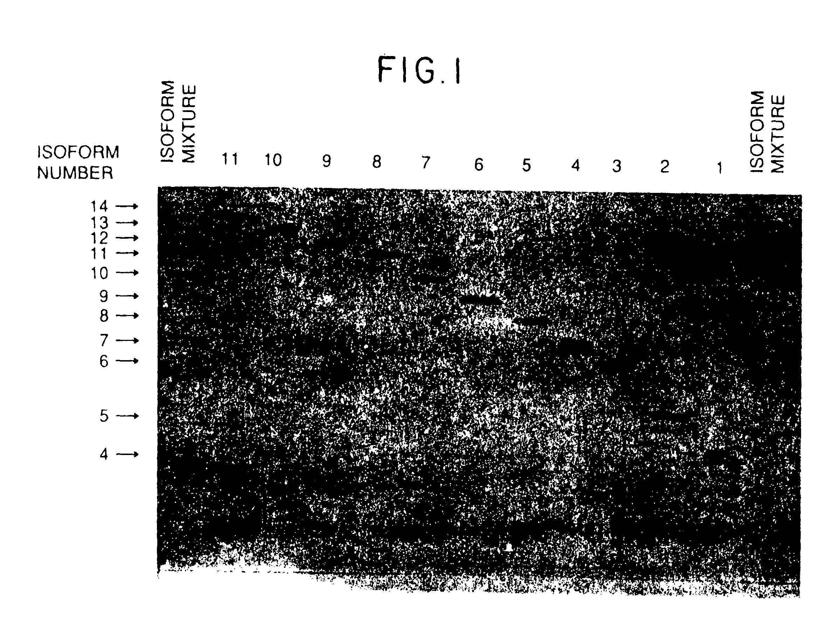

Figure 1 shows an analytical isoelectric focusing gel of the separate recombinant erythropoietin isoforms. Gel lanes 1-11 show isoforms ranging from less acidic (higher pI) in lane 1 to more acidic (lower pI), in lane 11. Purified recombinant erythropoietin containing a mixture of isoforms 9-14 is also shown in the far left and right lanes of the gel.

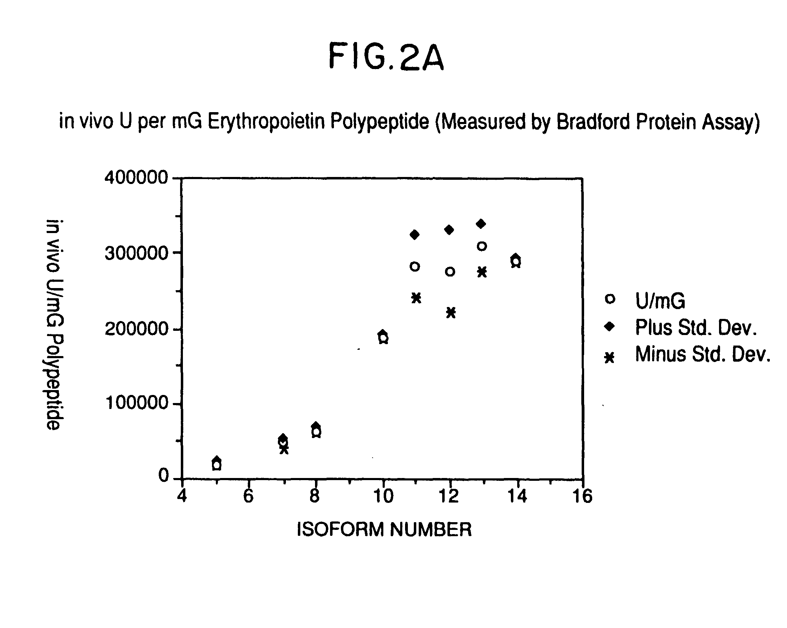

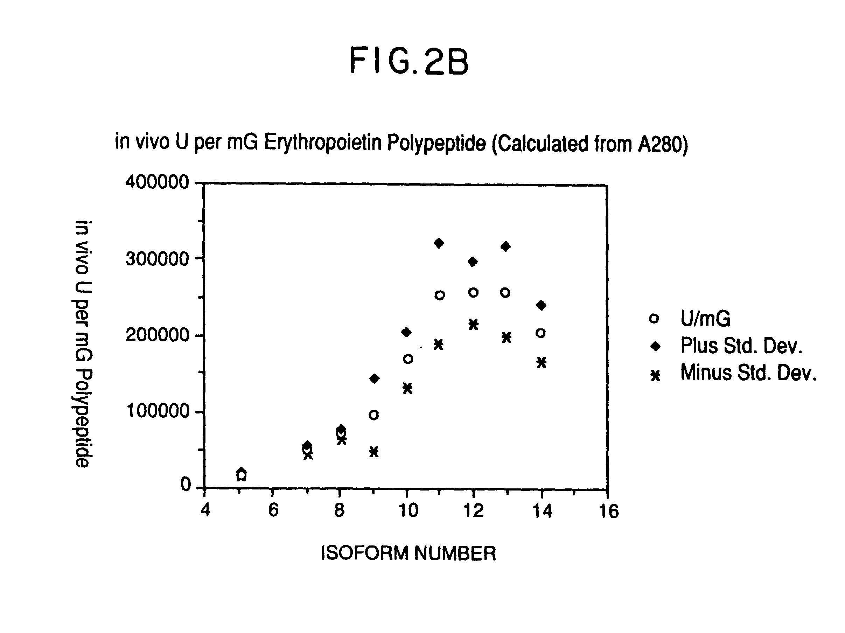

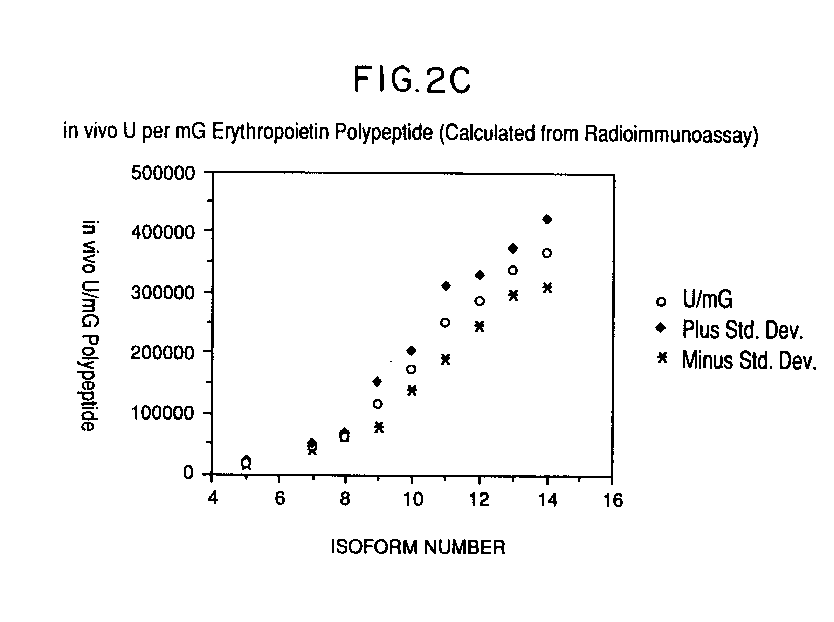

Figure 2 shows the relationship between the number of sialic acids per erythropoietin isoform and the in vivo specific activity of each isoform expressed as units per mg of erythropoietin polypeptide. In Figure 2A, the concentration of each erythropoietin isoform was determined by the Bradford protein assay; in 2B, the concentration was determined by absorbance at 280 nm, in 2C, the concentration was determined by RIA.

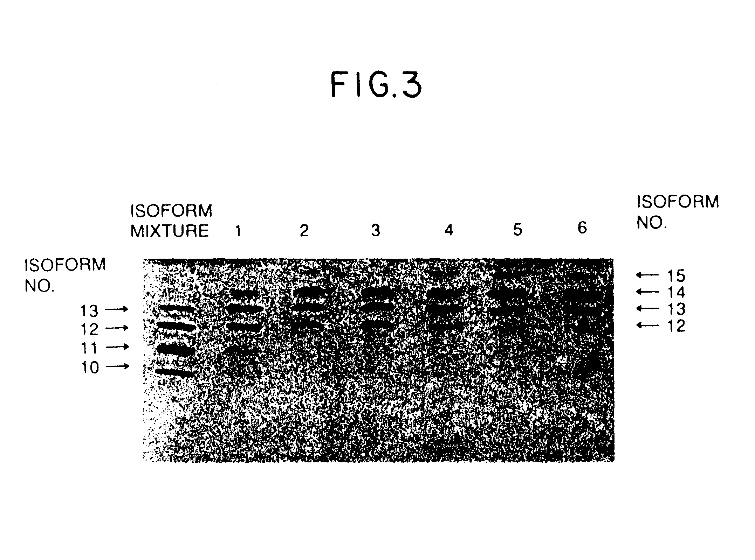

Figure 3 shows an analytical isoelectric focusing gel of defined mixtures of recombinant erythropoietin isoforms prepared by anion exchange chromatography under different conditions. Gel lanes 1-6 represent, respectively, erythropoietin isoforms eluted in a high salt wash after washing the Q-Sepharose fast flow column with 150 mM acetic acid, pH 4.7, 150 mM acetic acid (unbuffered), 200 mM acetic acid, pH 4.7, 250 mM acetic acid, pH 4.7, 300 mM acetic acid, pH 4.7 or 300 mM acetic acid (unbuffered). Purified recombinant erythropoietin containing a mixture of isoforms as obtained using procedures described in Example 2 of Lai et al., supra, except that DEAE-Agarose chromatography is replaced by Q-Sepharose chromatography, is also shown in the far left lane of the gel.

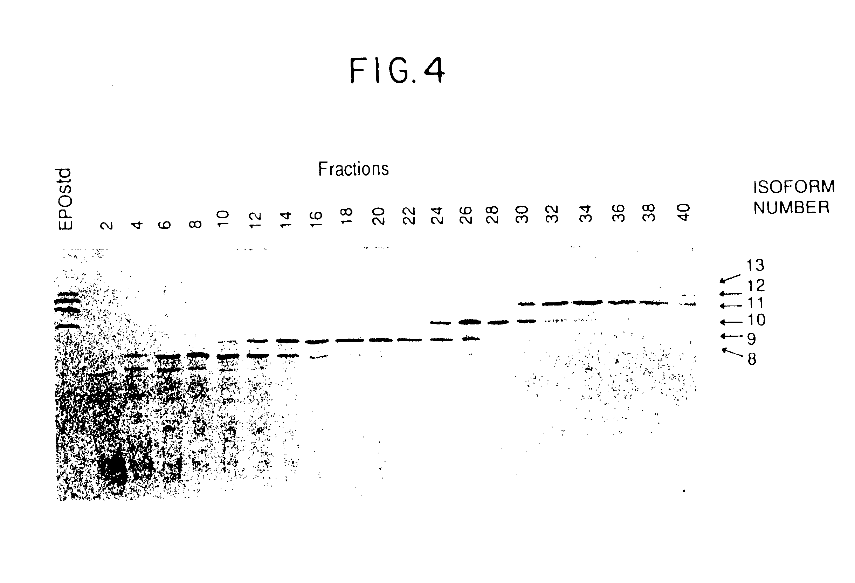

Figure 4 shows the separation of erythropoietin isoforms 8 to 12 achieved by subjecting cell conditioned medium applied to a column of Q-Sepharose to a gradient of decreasing pH and increasing ionic strength. Aliquots of even numbered fractions from Fraction 2 to Fraction 40 were subjected to analytical isoelectric focusing. Purified recombinant erythropoietin containing a mixture of isoforms obtained using procedures described in Example 2 of Lai et al. supra, except that DEAE-Agarose chromatography is replaced by Q-Sepharose chromatography, is also shown in the far left lane of the gel.

### Detailed Description of the Invention

According to the present invention, erythropoietin isoforms are provided. Isoelectric focusing (IEF) separates proteins on the basis of charge. When placed in a pH gradient and subjected to an electric field, proteins will migrate to the point at which they have no net charge and remain there. This is the isoelectric point (pl) of the protein. Each distinct band observed on IEF represents molecules that have a particular pl and therefore the same overall charge, and is termed an isoform. The term "erythropoietin isoform" as used herein refers to erythropoietin preparations having a single pI, and having the same amino acid sequence.

In a preferred embodiment the erythropoietin is the product of the expression of an exogenous DNA sequence that has been transfected into a non-human eucaryotic host cell, that is, in a preferred embodiment the erythropoietin is "recombinant erythropoietin". Recombinant erythropoietin is advantageously produced according to the procedures described in commonly owned Lin U.S. Patent 4,703,008 hereby incorporated by reference. Recombinant erythropoietin is advantageously purified according to the general procedures described in Example 2 of commonly owned Lai et al. U. S. Patent 4,667,016 hereby incorporated by reference, or alternatively the procedure described in Example 2 wherein DEAE-Agarose chromatography is replaced by Q-Sepharose chromatography. In the Q-Sepharose column modification, 55 mM NaCl replaces 25 mM NaCl in the buffer solution used to bring the column to neutral pH, and 140 mM NaCl replaces 75 mM NaCl in the buffer solution used to elute erythropoietin from the column. This material, when analyzed by sodium dodecyl sulfate polyacrylamide gel electrophoresis, migrates as a single species (i.e. band). When purified erythropoietin is subjected to IEF, multiple bands in the gel are apparent, indicating that different charged forms of the glycoprotein are present.

It has been found that discrete isoforms of recombinant erythropoietin having the amino acid sequence of urinary derived human erythropoietin correspond to erythropoietin molecules having from 1-14 sialic acids, and each isoform present in purified recombinant erythropoietin has an in vivo activity which is related to the number of sialic acids the isoform possesses. The term "erythropoietin", as used herein, includes naturally occurring erythropoietin, urinary derived human erythropoietin as well as non-naturally occurring polypeptides having an amino acid sequence and glycosylation sufficiently duplicative of that of naturally occurring erythropoietin to allow possession of in vivo biological properties of causing bone marrow cells to increase production of reticulocytes and red blood cells.

Crude preparations of erythropoietin have many isoforms but material purified for example, as in the Lai et al. patent supra Example 2, contains predominantly six isoforms when analyzed by IEF. In addition, at least one additional isoform of greater acidity has been detected using the chromatographic procedures described in Example 4. (This more acidic form, migrating at >14 sialic acids on an IEF gel may contain nonsialic acid negative charges as shown by the resistance of some of the charge to sialidase digestion). These isoforms differ from each other by sialic acid content. As shown in the Examples, this is demonstrated by isolating 10 of these isoforms by preparative IEF and determining the sialic acid content of five of them. Of the isoforms assayed for sialic acid content, it is found that the five isoforms contained either 9, 10, 11, 12 or 13 sialic acid residues.

There is a relationship between the relative in vivo specific activity of erythropoietin and number of sialic acid residues per erythropoietin molecule from the isoforms 5 through 11 (each isoform is designated herein by the number of sialic acids per erythropoietin molecule). Isoforms 11 through 14 have approximately the same relative in vivo specific activity. Isoforms 5-14 are assayed for in vivo activity by the exhypoxic polycythemic mouse bioassay and the amount of each isoform present is determined by Bradford protein assay, absorbance at 280 nm or by radioimmunoassay (RIA) for erythropoietin. RIA determinations (Egrie et al. Immunobiology 172, 213, (1986)), expressed as units/ml, are divided by 212,770 units/mg erythropoietin polypeptide, the average specific activity of purified erythropoietin as determined by RIA, to give protein concentrations of isolated isoforms or isoform mixtures expressed as mg erythropoietin polypeptide/ml. As shown in the Examples, the relative in vivo specific activities increase step-wise from isoform 5 to isoform 11 (see Table 2).

The in vivo specific activities referred to herein are measurements of relative in vivo specific activities and are not measurements of absolute in vivo specific activities. For the purposes of this application, the specific activities are used only to compare relative activities of isoforms that have been assayed using the same assay, using the same conditions including the same internal standard, the same type of animals, having the same analysis of the data used to calculate specific activity, the same assay for determining protein content. It is not intended that any in vivo specific activity value reported for any isoform represents an inherent or absolute value for that isoform.

The subject invention provides erythropoietin isoforms. The specific isoforms of erythropoietin obtained in accordance with the present invention, and their properties, may vary depending upon the source of the starting material. For example, the isoforms of urinary derived human erythropoietin are different than the isoforms of recombinant erythropoietin. In a preferred embodiment, the invention relates to an erythropoietin isoform having a specific number (i.e. a fixed number greater than 0) of sialic acids per erythropoietin molecule, said number selected from the group consisting of 1-14. Advantageously said number is 9, 10, 11, 12, 13, or 14. In another embodiment, said number is greater than 14, advantageously 16-23.

This invention also provides compositions comprising two or more erythropoietin isoforms. In one embodiment the compositions comprise a mixture of isoforms having greater than a predetermined number of sialic acids per erythropoietin molecule, e.g. greater than 11 sialic acids per erythropoietin molecule, or greater than 12 sialic acids per molecule, e.g. a mixture of isoforms 12, 13 and 14. In another embodiment the compositions comprise mixtures of isoforms having a predetermined number of sialic acids per erythropoietin molecule, e.g. less than 12, but greater than 8 sialic acids per molecule as in, for example, a mixture of isoforms 9, 10, and 11. The invention also provides for compositions of erythropoietin isoforms wherein the relative amounts of the isoforms are the same or different. For example, a mixture of isoforms 9, 10 and 11 could have the isoforms present in a variety of ratios such as 1:1:1, 2:3:1 or 20:20:1.

Advantageously, the compositions comprise mixtures of less than four isoforms, for example a mixture of isoforms 11, 12, and 13, or a mixture of 12 and 14, or a mixture of 7 and 13.

In order to produce mixtures of erythropoietin isoforms, this invention also provides methods of isolating selected erythropoietin isoforms simultaneously. These methods include isolation of individual isoforms by techniques such as preparative isoelectric focusing or preparation of mixtures of isoforms having a predetermined number of sialic acids per molecule (for example, greater than 11) by techniques such as ion exchange chromatography or chromatofocusing. All of these techniques have as their basis the separation of proteins according to charge.

In general, ion exchange chromatography and chromatofocusing involve application of either crude human erythropoietin (cell conditioned media) or purified material to a column resin under conditions that permit binding of some or all of the erythropoietin isoforms to the resin. For crude erythropoietin preparations, it is preferable to apply the protein to the column at about pH 7 while for purified preparations the protein can be applied to the column at pH 7 down to about pH 4. After washing the column with buffer at about pH 4, those erythropoietin isoforms that remain bound on the ion exchange column are eluted by increasing the pH and the salt concentration of the buffer or by applying a gradient of decreasing pH and increasing ionic strength at about pH 4. For chromatofocusing, the isoforms are eluted from the column by a gradient of decreasing pH or by washing the column with a high concentration of salt.

In one embodiment the invention relates to mammalian (e.g., Chinese Hamster Ovary, CHO) host cells which preferentially synthesize erythropoietin isoforms having greater than a specific number, e.g. greater than 10 sialic acids per molecule. Erythropoietin molecules have N-linked or O-linked oligosaccharides structures which can limit the sialic acid content of the molecule. For example, tetraantennary (four-branched) N-linked oligosaccharides most commonly provide four possible sites for sialic acid attachment while bi- and triantennary oligosaccharide chains, which can substitute for the tetraantennary form at asparagine-linked sites, commonly have at most only two or three sialic acids attached. O-linked oligosaccharides commonly provide two sites for sialic acid attachment. Thus, erythropoietin molecules can accommodate a total of 14 sialic acid residues provided all three N-linked oligosaccharides are tetraantennary. Mammalian cell cultures are screened for those cells that preferentially add tetraantennary chains to recombinant erythropoietin, thereby maximizing the number of sites for sialic acid attachment.

The N-linked oligosaccharides of urinary erythropoietin contain sialic acid in both an α 2,3 and an α 2,6 linkage to galactose (Takeuchi et al. J. Biol. Chem. 263, 3657(1988)). Typically the sialic acid in the α 2,3 linkage is added to galactose on the mannose α 1,6 branch and the sialic acid in the α 2,6 linkage is added to the galactose on the mannose α 1,3 branch. The enzymes that add these sialic acids (β-galactoside α 2,3 sialyltransferase and β-galactoside α 2,6 sialyltransferase) are most efficient at adding sialic acid to the mannose α 1,6 and mannose α 1,3 branches respectively.

Dihydrofolate reductase (DHFR) deficient Chinese Hamster Ovary (CHO) cells are a commonly used host cell for the production of recombinant glycoproteins including recombinant erythropoietin. These cells do not express the enzyme β-galactoside α 2,6 sialyltransferase and therefore do not add sialic acid in the α 2,6 linkage to N-linked oligosaccharides of glycoproteins produced in these cells. (Mutsaers et al. Eur. J. Biochem. 156, 651 (1986); Takeuchi et al. J. Chromatogr. 400, 207 (1987)). Consequently, recombinant erythropoietin produced in CHO cells lacks sialic acid in the 2,6 linkage to galactose (Sasaki et al. (1987), supra; Takeuchi et al. (1987), supra). In another embodiment of the subject invention, the erythropoietin used to produce the isoforms is made in CHO cells that are transfected with a functional β-galactoside α 2,6 sialyltransferase gene to give incorporation of sialic acid in α 2,6 linkage to galactose. See Lee et al. J. Biol. Chem. 264, 13848 (1989), hereby incorporated by reference, for a disclosure of techniques for creating modified CHO cells or other mammalian host cells.

Also disclosed by the invention are certain analogs of human erythropoietin. As used herein the phrase "analog of human erythropoietin" refers to erythropoietin with one or more changes in the amino acid sequence of human erythropoietin which result in an increase in the number of sites for sialic acid attachment. Analogs are generated by site-directed mutagenesis having additions, deletions, or substitutions of amino acid residues that alter sites that are available for glycosylation. Such analogs have a greater number of carbohydrate chains than human erythropoietin.

Analogs that result in increased biological activity are constructed by increasing the sialic acid content of the erythropoietin molecule. Analogs having levels of sialic acid greater than that found in human erythropoietin are generated by adding glycosylation sites which do not perturb the secondary or tertiary conformation required for biological activity. Advantageously, the analog of human erythropoietin has 1, 2 or 3 additional sites for N-glycosylation or O-glycosylation. For example, a leucine at position 69 is replaced by an asparagine to give the sequence Asn-Leu-Ser, which serves as a fourth site for N-glycosylation. Such a change can commonly provide up to four additional sialic acids per molecule. Examples of other changes that generate additional N- or O-glycosylation sites are alanines at positions 125 and 127 to asparagine and serine, respectively, alanine at position 125 to threonine and alanines at positions 124 and 125 to proline and threonine, respectively. As will be appreciated by those skilled in the art, the disclosure includes many other analogs of human erythropoietin having additional sites for glycosylation.

Also comprehended by the invention are pharmaceutical compositions comprising a therapeutically effective amount of a specific isoform or mixture of isoforms together with a suitable diluent, adjuvant and/or carrier useful in erythropoietin therapy. A "therapeutically effective amount" as used herein refers to that amount which provides therapeutic effect for a given condition and administration regimen. The administration of erythropoietin isoforms is preferably by parenteral routes. The specific route chosen will depend upon the condition being treated. The administration of erythropoietin isoforms is preferably done as part of a formulation containing a suitable carrier, such as human serum albumin, a suitable diluent, such as a buffered saline solution, and/or a suitable adjuvant. The required dosage will be in amounts sufficient to raise the hematocrit of patients and will vary depending upon the severity of the condition being treated, the method of administration used and the like.

The following examples are offered to more fully illustrate the invention, but are not to be construed as limiting the scope thereof. The erythropoietin standard used in the in vivo bioassays employed in the Examples is a recombinant erythropoietin standard that was standardized against a partially purified urinary erythropoietin standard. Thus, only relative in vivo specific activities are being measured. Also the in vivo specific activities are expressed in "units/ml", " "units/mg" and units/A₂₈₀" and not as "IU/ml", "IU/mg" and IU/A₂₈₀", because the erythropoietin standard employed has not been directly correlated to any existing international standard.

### Example 1: Isolation of Recombinant Erythropoietin Isoforms

Recombinant erythropoietin is produced as described in Lin, supra. Recombinant erythropoietin used as starting material for the first and third isoform isolations is purified according to the procedure described in Example 2 of commonly owned Lai et al., supra. Starting material for the second and fifth isoform isolation is purified according to Lai et al. supra using the modification of Q-Sepharose chromatography. These preparations contain a mixture of isoforms of recombinant erythropoietin having the same amino acid sequence as urinary derived human erythropoietin and contain predominantly isoforms 9 to 14. Starting material for the fourth isoform preparation is the material which elutes during the 5 mM acetic acid/1 mM glycine/6M urea wash of the anion exchange column in Example 2 of Lai et al. This fraction contains isoforms with less than or equal to 9 sialic acids and was further purified by gel filtration chromatography as described in Example 2 of Lai et al. prior to use in the preparative isoelectric focusing procedure. The sixth isoform preparation used as its starting material a purified preparation of recombinant erythropoietin having from 4 to 13 sialic residues. This material was purified as per Example 2 of Lai et al. except for a modification to the ion exchange column (elution of the recombinant erythropoietin with a sodium chloride gradient at pH 8.4 and omission of the acetic acid/urea wash) which results in retention of most of the isoforms present in the starting material.

Six different preparations of individual isoforms are carried out by preparative isoelectric focusing in a granulated gel bed (Ultrodex, LKB) essentially as per LKB Application Note 198. Pharmalyte (Pharmacia) 2.5-5 ampholytes (Pharmacia) are used and the gel bed contains 5 M urea.

In the first preparation, approximately 20 mg of recombinant erythropoietin in 6.8 ml of 20 mM sodium citrate/ 100 mM sodium chloride, pH 7.0 are applied to the gel and focused at 8 watts for approximately 16 hours. After isoelectric focusing, the isoform bands in the gel are visualized by a paper contact print of the gel bed. The print is made and then fixed by soaking in 3 changes (approximately 10 minutes each, room temperature) of fixing solution (40% methanol/10% acetic acid/10% TCA/3.5% sulfosalicylic acid), subjected to one change (approximately 10 minutes) of 40% methanol/10% acetic acid (30-60°C), stained for 15 minutes at 60°C in 0.125% Coomassie Blue R-250/40% methanol/10% acetic acid, and then destained in 7.5% methanol/10% acetic acid in order to visualize the separated isoforms. The region of the granulated gel bed containing the isoforms (∼50% of the resin) is removed, water is added (∼16 ml), and the slurry is poured into a 5.5 x 24.5 inch tray and evaporated to ∼40 g net weight. This preparation is focused for a second time and a contact print of the gel bed is made as before. The portion of gel containing each of the six discernible isoforms is removed from the gel bed.

In order to elute the isoforms from the gel, a solution containing 10 mM Tris-HC1, pH 7.0/ 5 mM Chaps is added to each isoform to generate a slurry. The slurries are placed in small columns and washed with the Tris-Chaps buffer. The flow throughs are collected and applied separately to small columns (open column configuration) containing Vydac C4 reversed phase resin equilibrated in 20% ethanol/10 mM Tris-HCl, pH 7.0. The columns are developed stepwise with 20% ethanol/10 mM Tris-HCl, pH 7.0, 35% ethanol/10 mM Tris-HCl, pH 7.0, and 65% ethanol/10 mM Tris-HCl, pH 7.0. The fraction eluting at 65% ethanol/10 mM Tris is diluted 1:1 with 10 mM Tris-HCl, pH 7.0 and subjected to concentration and then buffer exchanged to 10 mM Tris-HCl, pH 7.0 using a Centricon-10 (Amicon) microconcentrator. Analytical isoelectric focusing of this preparation is performed essentially as described in LKB technical note 250 using Servalyte 3-5 ampholines (Serva) in a polyacrylamide gel containing 5 M urea.

In a second preparation, approximately 26 mg of recombinant erythropoietin in 6.5 ml of deionized water are applied to the gel and focused at 2.5 watts for 35 minutes and 10 watts for approximately 17 hours. The bands of focused protein, which are visible in the gel bed, are removed as 11 different pools. Each pool is brought to about 7.5 ml with deionized water and 20 ml of each of the resulting pool supernatants is subjected to analytical isoelectric focusing as described above. To each of the pools is added 5 ml of 1.5 M Tris-HCl, pH 8.8 and the slurries are each placed in small columns and the liquid phase allowed to flow through. The resin is washed with approximately three volumes of 0.5 M Tris-HCl, pH 7 and the rinse solution is combined with the flow through. The eluants are concentrated and buffer exchanged to 20 mM sodium citrate/ 100 mM sodium chloride, pH 7.0 using Amicon disposable ultrafiltration devices having a 10,000 dalton molecular weight cutoff. The concentrated solutions (approximately 0.5 ml) are then passed through a 0.22 micron cutoff cellulose acetate filter. Based upon analytical isoelectric focusing, five pools are found to contain predominantly the single isoforms 10, 11, 12, 13 and 14.

In a third preparation, approximately 30 mg of recombinant erythropoietin in 21.8 ml of distilled water is applied to the gel and focused at 2 watts for 25 minutes, 10 watts for 20 hours and 15 watts for 15 minutes. Protein bands corresponding to the individual isoforms are observed visually and removed from the gel bed. Distilled water is added to gel-isolated isoforms to generate a slurry and the resulting supernatants are analyzed by analytical isoelectric focusing. An equal volume of 1 M Tris-HCl, pH 7.2 is added to each slurry, the suspensions are placed into separate small columns, and the liquid phase is allowed to flow through the column to elute the isoforms. Each flow through is concentrated and buffer exchanged to 20 mM sodium citrate/ 100 mM sodium chloride, pH 7.0 using Amicon disposable ultrafiltration devices having a 10,000 dalton molecular weight cutoff. An analytical isoelectric focusing gel revealed that pools containing predominantly the single isoforms 9, 10, 11, 12, 13 and 14 were obtained.

A fourth isoform preparation used as its starting material erythropoietin containing isoforms 3-9 (prepared as described above). Prior to preparative isoelectric focusing carried out essentially as described for preparations 1-3 above, the ampholytes (Pharmalyte 2.5-5) were pre-fractionated in a Rotofor (Bio-Rad, Richmond, CA) liquid phase isoelectric focusing cell to yield an ampholyte range more suitable for the lower isoelectric points of the starting material. The prefractionation was carried out by mixing 6.7 mL of Pharmalyte 2.5-5 with 15 g of urea and adding purified water to bring the volume to 50 mL. This mixture was fractionated in the Rotofor at 10 Watts, 1°C, for 5 1/2 hours using 0.1 M phosphoric acid and 0.1 M sodium hydroxide as the anolyte and catholyte, respectively. The ampholyte fractions having measured pHs of between 4.5 and approximately 6 were used in the flat-bed isoelectric focusing.

Ampholytes were removed from the isoforms using a Centrieluter (Amicon, Danvers, MA) and a 10,000 MW cutoff Centricon (Amicon) using the following parameters: 0.18 Tris buffer pH 8.8, 100 Volts, 25-30 mA, for 3 hours. The isoforms were then buffer exchanged into 0.1 M sodium chloride by gel filtration using Sephadex G-25 (Pharmacia). Analytical isoelectric focusing of the five resulting pools showed them to contain isoforms 4,5,6,7, and 8. Isoform 4 ran as several bands, indicating that it may have undergone some degradation.

The fifth isoform preparation was modified by the addition of a pre-focusing step to the flat bed isoelectric focusing procedure. In this modification, the protein was not added to the ampholyte/urea/gel mixture prior to electrophoresis but was added to the isoelectric focusing apparatus following generation of the pH gradient in the gel bed. Following prefo-cusing for 75 minutes (1500 volt-hrs) the section of gel bed from 2.25-4.25 cm from the cathode was removed, mixed with the erythropoietin solution, and added back to the gel bed. Following isoelectric focusing, isoforms 10,11,12,13, and 14 were eluted from the gel bed and separated from the ampholytes by ultrafiltration using Centricon-10 (Amicon) devices.

The pre-focusing modification was undertaken to make the ultraviolet absorbance characteristics of the isoform preparations more similar to that of the starting recombinant erythropoietin. This improvement in spectral characteristics can be seen in the ratio of absorbance at 280 and 260 nm for the isolated isoforms. The average ratio of absorbance at 280 nm to that at 260 nm (A280/A260) for isoforms from preparations 2 and 3 (non-prefocused) is 1.36 ± 0.11 while the average A280/A260 ratio for preparations 5 and 6 (pre-focused) is 1.68 ± 0.20. When isoform #14 is excluded from the calculation, the average A280/A260 ratios are 1.39 ± 0.11 and 1.74 ± 0.09 for preparations 2 & 3 and 5 & 6, respectively. (Isoform 14 may have the most atypical spectrum because it is present in the smallest amounts and is thus more subject to interferences by trace contamination by ampholyte components or because it is nearest to the electrode during the flat bed isoelectric focusing procedure). The average A280/A260 ratio for recombinant erythropoietin prepared according to Example 2 of Lai et al. (modified as described earlier by using Q-Sepharose as the anion exchange resin) is 1.91 ± 0.04.

As described above, the starting material for isoform preparation #6 was a recombinant erythropoietin preparation containing isoforms 4-13. The ampholytes were pre-focused in the Rotofor apparatus as per the fourth preparation. Ampholyte fractions having measured pHs of between 3.7 and 4.8 were used for the flat bed isoelectric focusing. The flat bed was pre-focused as in run #5 and isoforms 9,10,11,12 and 13 were obtained after ultrafiltration (Centricon-10) to remove carrier ampholytes.

### Example 2: Sialic Acid Content of Recombinant Erythropoietin Isoforms

The isoforms isolated as described in Example 1 and erythropoietin purified according to procedures described in Lai et al., supra (mixture of isoforms 9 to 14) are buffer exchanged into 0.10-0.15 M sodium chloride and analyzed for sialic acid content by a modification of the procedure of Jourdian et al. J. Biol. Chem. 246, 430 (1971). The sialic acid residues are cleaved from the glycoproteins by hydrolysis with 0.35 M sulfuric acid at 80°C for 30 minutes and the solutions are neutralized with sodium hydroxide prior to analysis. In order to estimate the amount of erythropoietin protein present, a Bradford protein assay (Bradford Anal. Biochem. 72, 248 (1976)) using recombinant erythropoietin having the amino acid sequence of human erythropoietin as standard is performed using the assay reagents and the micro-method procedure supplied by Bio-Rad. The results, expressed as moles of sialic acids per mole of erythropoietin, are shown in Table 1. Isoforms are designated according to the number of sialic acids per molecule and range from least acidic (Isoform 9) to most acidic (Isoform 13). Isoforms 9-13 are shown in gel lanes 6-10 of Figure 1. Quantities of Isoform 14 are insufficient to accurately measure the sialic acid content. The sialic acid content of this isoform is inferred from its migration on IEF gels relative to other isoforms. The sialic acid content of isoforms 5-8 (preparation #4) has not been measured but is likewise inferred from their migration on IEF gels.

**TABLE 1**

| ERYTHROPOIETIN ISOFORM | MOLES SIALIC ACID/MOLE ERYTHROPOIETIN |
|---|---|
| Isoform 13 | 12.9 ± 0.5 |
| Isoform 12 | 11.8 ± 0.2 |
| Isoform 11 | 11.0 ± 0.2 |
| Isoform 10 | 9.8 ± 0.3 |
| Isoform 9 | 8.9 ± 0.6 |
| Isoform Mixture (9-14) | 11.3 ± 0.2 |

### Example 3: Activity of Recombinant Erythropoietin Isoforms

The isoforms isolated as described in Example 1 are assayed by absorbance at 280 nm, by Bradford protein assay and by RIA for erythropoietin to determine the amount of recombinant erythropoietin present. The exhypoxic polycythemic mouse bioassay (Cotes et al. Nature 191, 1065 (1961)) is used to determine the relative in vivo biological activity. Quantitation of the amount of erythropoietin protein present using a radioimmunoassay for erythropoietin produced results having higher relative in vivo specific activity for certain isoforms because of an apparent decreased immunoreactivity of isoforms containing large amounts of sialic acid leading to an underestimation of the erythropoietin concentration and thus an overestimation of the relative in vivo specific activity for the most negative isoforms. Mouse bioassay determinations, expressed as units/ml, are divided by the corresponding protein concentrations to give in vivo specific activities expressed as units/mg erythropoietin polypeptide. These specific activities are shown in Table 2.

In Table 2, "n" is the number of independent isoform preparations which contribute to the specific activity value. In most cases several in vivo assays were performed on each isoform preparation. The same in vivo data contribute to the specific activity calculations for all three columns, units/mg erythropoietin polypeptide was determined by the absorbance at 280 nm, from radioimmunoassay potencies, or from Bradford protein assay results. Purified recombinant erythropoietin containing isoforms 9-14 was used as the standard in the Bradford protein assay. "n" may be less for the calculation made using the Bradford protein assay as some preparations were no longer available at the time the Bradford assays were performed.

Erythropoietin purified according to the procedures described in Lai et al., supra and containing a mixture of isoforms 9 to 14 is used as a standard for the RIAs and in vivo assays.

The relative specific activities expressed as units/mg erythropoietin polypeptide can be converted to units/A₂₈₀ by multiplying by 0.807 mg erythropoietin polypeptide/A₂₈₀. The conversion factor is derived by multiplying the extinction coefficient of erythropoietin (1.345 mg/A₂₈₀) by the protein content of the erythropoietin glycoprotein (about 60% by weight, Davis et al. Biochemistry 26, 2633 (1987)) to obtain mg erythropoietin polypeptide/A₂₈₀ (i.e., 1.345 mg erythro-poietin/A₂₈₀ x 0.60 mg polypeptide/mg erythropoietin = 0.807 mg polypeptide/A₂₈₀). In addition, specific activities expressed as units/mg erythropoietin polypeptide can be multiplied by the factor 0.60 mg polypeptide/mg erythropoietin glycoprotein to give specific activities expressed as units/mg erythropoietin glycoprotein.

**TABLE 2**

| Isoform | U/mG Polypeptide (Bradford Protein Assay) | n | U/mG Polypeptide (From A280) | n | U/mG Polypeptide (From RIA) | n |
|---|---|---|---|---|---|---|
| 14 | 289,400 ± 3,100 | 2 | 205,800 ± 37,700 | 2 | 366,700 ± 55,900 | 2 |
| 13 | 307,600 ± 30,600 | 4 | 258,700 ± 59,500 | 5 | 337,200 ± 40,200 | 5 |
| 12 | 275,200 ± 55,600 | 4 | 258,400 ± 41,700 | 5 | 287,700 ± 42,600 | 5 |
| 11 | 282,700 ± 41,100 | 3 | 255,800 ± 67,300 | 4 | 251,400 ± 62,700 | 4 |
| 10 | 188,000 ± 1,900 | 1 | 170,300 ± 34,500 | 3 | 171,900 ± 31,600 | 3 |
| 9 | - | | 96,600 ± 46,700 | 2 | 113,600 ± 39,600 | 2 |
| 8 | 65,200 ± 3,800 | 1 | 70,600 ± 4,100 | 1 | 61,000 ± 3,500 | 1 |
| 7 | 46,200 ± 5,800 | 1 | 50,300 ± 6,300 | 1 | 42,800 ± 5,400 | 1 |
| 5 | 16,600 ± 1,700 | 1 | 18,300 ± 1,900 | 1 | 15,500 ± 1,600 | 1 |

The data in Table 2 are also presented graphically in Figures 2A, 2B and 2C. These data show that the relative in vivo activity of erythropoietin increases as a function of sialic acid content up until isoform #11. Isoforms 11-14 have essentially the same relative in vivo bioactivity. (This is most apparent when the concentration of isoform 14 is expressed using the Bradford assay value. The Bradford value may be more accurate for isoform 14 because of the generally low levels obtained and the resulting difficulty in determination by A₂₈₀ and the most apparent decreased reactivity in the RIA of very negative forms discussed previously). The greater relative in vivo specific activity of erythropoietin isoforms having more sialic acid is most likely due to a longer circulating half-life of these forms. Isoforms 9 and 13 were labeled with radioactive iodine (¹²⁵I) and their rate of clearance in rats was determined. The half-life in circulation was significantly longer for isoform 13 than for isoform 9.

### Example 4: Selection of Recombinant Ervthropoietin Isoform Mixtures by O-Sepharose Chromatography

Cell conditioned media from the production of recombinant erythropoietin according to the procedures described in Lin, supra are concentrated and diafiltered against 10 mM Tris, pH 7.2. Protein concentration is determined by the Bradford microprotein assay using bovine serum albumin as a standard. 19.6 ml of the solution containing 40 mg of total protein is made 20 µM in CuS0₄, filtered through a 0.45 micron cutoff filter and loaded onto a 4 ml bed volume (1.05 cm height x 2.2 cm diameter) column packed with Q Sepharose Fast Flow (Pharmacia) which has been equilibrated with 10 mM Tris, pH 6.8 to 7.0 at 4°C. After sample application, the column is washed with two column volumes of the same buffer. The column flow rate is about 1 ml/min. Six separate columns are set up using this procedure to select defined erythropoietin isoform mixtures.

Columns are washed with 6 to 9 column volumes of a low pH buffer consisting of: Column #1, 150 mM acetic acid, 1 mM glycine, 20 µM CuSO₄, 6 M urea adjusted to pH 4.7 with NaOH; Column #2, 200 mM acetic acid, 1 mM glycine, 20 µM CuSO₄, 6 M urea adjusted to pH 4.7 with NaOH; Column #3, 250 mM acetic acid, 1 mM glycine, 20 µM CuSO₄, 6 M urea adjusted to pH 4.7 with NaOH; Column #4, 300 mM acetic acid, 1 mM glycine, 20 µM CuSO₄, 6 M urea adjusted to pH 4.7 with NaOH; Column #5, 150 mM acetic acid, 1 mM glycine, 20 µM CuSO₄, 6 M urea; Column #6, 300 mM acetic acid, 1 mM glycine, 20 µM CuSO₄, 6 M urea. The pH of the columns is increased to approximately pH 7 by washing each one with 8 to 11 column volumes of 10 mM Tris-HCl, 55 mM NaCl, 20 M CuSO₄, pH 7. The defined erythropoietin isoform mixtures are eluted from the columns by washing with 10 mM Tris-HCl, 140 mM NaCl, 20 µM CuSO₄, pH 7.0.

The eluted isoform pools from each column are concentrated and solvent exchanged into water using an Amicon Centricon-10 microconcentrator. The results of analytical isoelectric focusing of these concentrated pools are shown in Figure 3. Gel lanes 1-6 represent defined erythropoietin isoform mixtures eluted from column 1,5,2,3,4 and 6 respectively. The "isoform mixture" shown in the far left gel lane of Figure 3 represents cell media which is applied to a Q-Sepharose column as described above, the column is washed with 5 mM acetic acid, 1 mM glycine, 20 µM CuSO₄, 6M urea, and the erythropoietin isoform mixture is eluted from the column using the procedures described above. This eluted mixture of isoforms is further purified according to the procedures described in Lai et al., supra prior to analytical isoelectric focusing.

### Example 5: Fractionation of Recombinant Erythropoietin lsoforms Using a Low pH Gradient on O-Sepharose

In another procedure, erythropoietin isoforms are separated using a gradient of decreasing pH and increasing ionic strength. The concentrated diafiltered erythropoietin containing media is loaded to a column of Q-Sepharose at a ratio of approximately 40 mg total protein/mL gel. The column is then washed with approximately two column volumes of 10 mM Tris HCl, pH 7.0 and then approximately 10 column volumes of 2 mM acetic acid/1 mM glycine/20 µM CuSO₄/6 M urea (pH approximately 4.8) to remove contaminating proteins and erythropoietin isoforms containing less than approximately 7 sialic acid residues. Isoforms containing from approximately 8 to approximately 12 sialic acids are eluted from the column using a gradient starting at approximately 2 mM acetic acid in 6 M urea/1 mM glycine/20 µM CuSO₄ and running to 40 mM acetic acid/6 M urea/1 mM glycine/20 µM CuSO₄ (pH approximately 4). The total volume of the gradient is approximately 40 column volumes and fractions of approximately one column volume each are collected into vessels containing a volume of Tris buffer sufficient to bring the pH into the range of 6-8.5 so as to avoid long term exposure of the collected fractions to low pH. Aliquots of the fractions are subjected to analytical isoelectric focusing to monitor the separation. Figure 4 shows the separation of isoforms 8-11 which may be achieved by this procedure. Isoforms 12-14 which remain bound to the column at the end of the gradient are eluted by washing with a buffer consisting of 10 mM TrisHCl, 140 mM NaCl, 20 µM CuSO₄ (pH 7.0). The isoforms (separated during the gradient or eluted by the sodium chloride solution) are freed of contaminating proteins by reverse phase chromatography followed by gel filtration chromatography as described in Example 2 of Lai et al.

## Claims (Claims for the following Contracting State(s): AT, BE, CH, DE, DK, FR, GB, IT, LI, LU, NL, SE)

1. An isolated biologically active erythropoietin isoform having a single isoelectric point and having a specific number of sialic acids per erythropoietin molecule, said number being selected from the group consisting of 1 to 14.

2. An erythropoietin isoform according to Claim 1 wherein said isoform is the product of the expression of an exogenous DNA sequence in a non-human eucaryotic host cell.

3. An erythropoietin isoform according to Claim 1 wherein said isoform comprises erythropoietin having the amino acid sequence of 1-165 or 1-166 human erythropoietin.

4. An erythropoietin isoform according to Claim 1 having 14 sialic acids per erythropoietin molecule.

5. An erythropoietin isoform according to Claim 1 having 13 sialic acids per erythropoietin molecule.

6. An erythropoietin isoform according to Claim 1 having 10 sialic acids per erythropoietin molecule.

7. An erythropoietin isoform according to Claim 2 wherein said eucaryotic host cell is a CHO cell.

8. A pharmaceutical composition comprising a therapeutically effective amount of said erythropoietin isoform of Claim 1 and a pharmaceutically acceptable diluent, adjuvant or carrier.

9. A composition consisting essentially of two or three erythropoietin isoforms according to Claim 1.

10. A composition according to Claim 9 consisting essentially of erythropoietin isoforms having less than 12 sialic acids per molecule.

11. A composition according to Claim 10 consisting essentially of erythropoietin isoforms having 9, 10 and 11 sialic acids per erythropoietin molecule.

12. A composition according to Claim 9 consisting essentially of erythropoietin isoforms having greater than 11 sialic acids per molecule.

13. A composition as in Claim 12 consisting essentially of erythropoietin isoforms having 13 and 14 sialic acids per molecule.

14. Erythropoietin consisting essentially of biologically active erythropoietin molecules having an identical number of sialic acids per molecule, said number being selected from the group consisting of 1 to 14.

15. Erythropoietin according to Claim 14 having 14 sialic acids per erythropoietin molecule

16. Erythropoietin according to Claim 14 having 13 sialic acids per erythropoietin molecule.

17. Erythropoietin according to Claim 14 having 10 sialic acids per erythropoietin molecule.

18. Erythropoietin according to Claim 14 wherein said molecule is the product of the expression of an exogenous DNA sequence in a non-human eucaryotic host cell.

19. Erythropoietin according to Claim 13 wherein said erythropoietin has the amino acid sequence of human erythropoietin.

20. A pharmaceutical composition comprising a therapeutically effective amount of erythropoietin of Claim 14 and a pharmaceutically acceptable diluent, adjuvant or carrier.

21. A method of preparing an erythropoietin isoform according to Claim 1 comprising the steps of: subjecting a purified erythropoietin to preparative isoelectric focusing, and eluting a single isoform from the gel.

22. A method of preparing a mixture of erythropoietin isoforms having a predetermined number of sialic acids per molecule, said number being greater than 11, comprising subjecting material containing erythropoietin to ion exchange chromatography.

23. A method of preparing a mixture of erythropoietin isoforms having a predetermined number of sialic acids per molecule, said number being greater than 11, comprising subjecting a material containing erythropoietin to chromatofocusing.

24. The composition according to Claim 9 for use in a method of increasing hematocrit levels in mammals.

25. A method for obtaining an erythropoietin composition having a predetermined number of scalic acids per molecule comprising preparing a mixture of two or more erythropoietin isoforms according to claim 1,
wherein said mixture consists essentially of at least two isoforms having less than 12 sialic acids per molecule, or
wherein said mixture consists essentially of erythropoietin isoforms having 9, 10 and 11 sialic acids per molecule, or
wherein said mixture consists essentially of at least two isoforms having greater than 11 sialic acids per molecule, or wherein said mixture consists essentially of erythropoietin isoforms having 13 and 14 sialic acids per molecule.

## Claims (Claims for the following Contracting State(s): ES, GR)

1. A method of preparing a biologically active erythopoietin isoform, which method comprises isolating a biologically active erythropoietin isoform having a single isoelectric point and having a specific number of sialic acids per erythropoietin molecule, said number being selected from the group consisting of 1 to 14.

2. A method according to Claim 1, wherein said isoform is produced by the expression of an exogenous DNA sequence in a non-human eucaryotic host cell.

3. A method according to Claim 1, wherein said isoform comprises erythropoietin having the amino acid sequence of 1-165 or 1-166 human erythropoietin.

4. A method according to Claim 1, wherein said isoform has 14 sialic acids per erythropoietin molecule.

5. A method according to Claim 1, wherein said isoform has 13 sialic acids per erythropoietin molecule.

6. A method according to Claim 1, wherein said isoform has 10 sialic acids per erythropoietin molecule.

7. A method according to Claim 2, wherein said eucaryotic host cell is a CHO cell.

8. A method of preparing a pharmaceutical composition, which method comprises combining a therapeutically effective amount of an erythropoietin isoform prepared according to Claim 1, with a pharmaceutically acceptable diluent, adjuvant or carrier.

9. A method of preparing erythropoietin, which method essentially comprises preparing biologically active erythropoietin molecules having an identical number of sialic acids per molecule, said number being selected from the group consisting of 1 to 14.

10. A method according to Claim 9, in which there are 14 sialic acids per erythropoietin molecule.

11. A method according to Claim 9, in which there are 13 sialic acids per erythropoietin molecule.

12. A method according to Claim 9, in which there are 10 sialic acids per erythropoietin molecule.

13. A method according to Claim 9, wherein said molecule is produced by the expression of an exogenous DNA sequence is a non-human eucaryotic host cell.

14. A method of preparing a pharmaceutical composition, which method comprises combining a therapeutically effective amount of erythropoietin prepared according to the method of Claim 9 with a pharmaceutically acceptable diluent, adjuvant or carrier.

15. A method of preparing an erythropoietin isoform according to Claim 1, comprising the steps of: subjecting a purified erythropoietin to preparative isoelectric focusing, and eluting a single isoform from the gel.

16. A method of preparing a mixture of erythropoietin isoforms having a predetermined number of sialic acids per molecule, said number being greater than 11, comprising subjecting material containing erythropoietin to ion exchange chromatography.

17. A method of preparing a mixture of erythropoietin isoforms having a predetermined number of sialic acids per molecule, said number being greater than 11, comprising subjecting a material containing erythropoietin to chromatofocusing.

18. A method for obtaining an erythropoietin composition having a predetermined number of sialic acids per molecule comprising preparing a mixture of two or more erythropoietin isoforms prepared according to the method of Claim 1,
wherein said mixture consists essentially of ar least-two isoforms having less than 12 sialic acids per molecule, or
wherein said mixture consists essentially of erythropoietin isoforms having 9, 10 and 11 sialic acids per molecule, or
wherein said mixture consists essentially of at least two isoforms having greater than 11 sialic acids per molecule.

19. method as in Claim 18 wherein said mixture consists essentially of erythropoietin isoforms having 13 and 14 sialic acids per molecule.

20. A method according to Claim 18, for use in a method of increasing hematocrit levels in mammals.

21. A method according to Claim 19, wherein said erythropoietin has the amino acid sequence of human erythropoietin.

## Patentansprüche (Patentansprüche für folgende(n) Vertragsstaat(en): AT, BE, CH, DE, DK, FR, GB, IT, LI, LU, NL, SE)

1. Eine isolierte biologisch aktive Erythropoietin-Isoform, mit einem einzigen isoelektrischen Punkt und mit einer spezifischen Anzahl von Sialinsäuren pro Erythropoietin-Molekül, wobei besagte Anzahl ausgewählt ist aus der Gruppe, die aus 1 bis 14 besteht.

2. Eine Erythropoietin-Isoform nach Anspruch 1, wobei besagte Isoform das Produkt des Expression einer exogenen DNA-Sequenz in einer nicht-menschlichen eukaryontischen Wirtszelle ist.

3. Eine Erythropoietin-Isoform nach Anspruch 1, wobei besagte Isoform Erythropoietin umfaßt, das die Aminosäuresequenz von 1-165- oder 1-166-Human-Erythropoietin aufweist.

4. Eine Erythropoietin-Isoform nach Anspruch 1, mit 14 Sialinsäuren pro Erythropoietin-Molekül.

5. Eine Erythropoietin-Isoform nach Anspruch 1, mit 13 Sialinsäuren pro Erythropoietin-Molekül.

6. Eine Erythropoietin-Isoform nach Anspruch 1, mit 10 Sialinsäuren pro Erythropoietin-Molekül.

7. Eine Erythropoietin-Isoform nach Anspruch 2, wobei besagte eukaryontische Wirtszelle eine CHO-Zelle ist.

8. Eine pharmazeutische Zusammensetzung, die eine therapeutische wirksame Menge an besagter Erythropoietin-Isoform von Anspruch 1 und ein pharmazeutische annehmbares Verdünnungsmittel, Adjuvans oder Trägermittel umfaßt.

9. Eine Zusammensetzung, die im wesentlichen aus zwei ider drei Erythropoietin-Isoformen nach Anspruch 1 besteht.

10. Eine Zusammensetzung nach Anspruch 9, die im wesentlichen aus Erythropoietin-Isoformen mit weniger als 12 Sialinsäuren pro Molekül besteht.

11. Eine Zusammensetzung nach Anspruch 10, die im wesentlichen aus Erythropoietin-Isoformen mit 9, 10 und 11 Sialinsäuren pro Molekül besteht.

12. Eine Zusammensetzung nach Anspruch 9, die im wesentlichen aus Erythropoietin-Isoformen mit mehr als 11 Sialinsäuren pro Molekül besteht.

13. Eine Zusammensetzung nach Anspruch 12, die im wesentlichen aus Erythropoietin-Isoformen mit 13 und 14 Sialinsäuren pro Molekül besteht.

14. Erythropoietin, das im wesentlichen aus biologisch aktiven Erythropoietin-Molekülen mit einer identischen Anzahl von Sialinsäuren pro Molekül besteht, wobei besagte Anzahl ausgewählt ist aus der Gruppe, die aus 1 bis 14 besteht.

15. Erythropoietin nach Anspruch 14, mit 14 Sialinsäuren pro Erythropoietin-Molekül.

16. Erythropoietin nach Anspruch 14, mit 13 Sialinsäuren pro Erythropoietin-Molekül.

17. Erythropoietin nach Anspruch 14, mit 10 Sialinsäuren pro Erythropoietin-Molekül.

18. Erythropoietin nach Anspruch 14, wobei besagtes Molekül das Produkt der Expression einer exogenen DNA-Sequenz in einer nicht-menschlichen eukaryontischen Wirtszelle ist.

19. Erythropoietin nach Anspruch 13, wobei besagtes Erythropoietin die Aminosäuresequenz von Human-Erythropoietin aufweist.

20. Eine pharmazeutische Zusammensetzung, die eine therapeutische wirksame Menge an Erythropoietin von Anspruch 14 und ein pharmazeutisch annehmbares Verdünnungsmittel, Adjuvans oder Trägermittel umfaßt.

21. Ein Verfahren zur Herstellung einer Erythropoietin-Isoform nach Anspruch 1, welches die Schritte umfaßt, daß ein gereinigtes Erythropoietin präparativer isoelektrischer Fokussierung unterzogen wird und eine einzelne Isoform aus dem Gel eluiert wird.

22. Ein Verfahren zur Herstellung einer Mischung von Erythropoietin-Isoformen mit einer vorbestimmten Anzahl von Sialinsäuren pro Molekül, wobei besagte Anzahl größer als 11 ist, welches umfaßt, daß Material, welches Erythropoietin enthält, einer Ionenaustauschchromatographie unterzogen wird.

23. Ein Verfahren zur Herstellung einer Mischung von Erythropoietin-Isoformen mit einer vorbestimmten Anzahl von Sialinsäuren pro Molekül, wobei besagte Anzahl größer als 11 ist, welches umfaßt, daß ein Material, das Erythropoietin enthält, einer Chromatofokussierung unterzogen wird.

24. Die Zusammensetzung nach Anspruch 9 zur Verwendung in einem Verfahren zur Erhöhung der Hämatokritgehalte in einem Verfahren zur Erhöhung der Hämatokritgehalte in Säugetieren.

25. Ein Verfahren zum Erhalten einer Erythropoietin-Zusammensetzung mit einer vorbestimmten Anzahl von Sialinsäuren pro Molekül, welches umfaßt, daß eine Mischung aus zwei oder mehr Erythropoietin-Isoformen nach Anspruch 1 hergestellt wird, wobei besagte Mischung im wesentlichen aus wenigstens zwei Isoformen mit weniger als 12 Sialinsäuren pro Molekül besteht oder wobei besagte Mischung im wesentlichen aus Erythropoietin-Isoformen mit 9, 10 und 11 Sialinsäuren pro Molekül besteht oder wobei besagte Mischung im wesentlichen aus wenigstens zwei Isoformen mit mehr als 11 Sialinsäuren pro Molekül besteht oder wobei besagte Mischung im wesentlichen aus Erythropoietin-Isoformen mit 13 und 14 Sialinsäuren pro Molekül besteht.

## Patentansprüche (Patentansprüche für folgende(n) Vertragsstaat(en): ES, GR)

1. Ein Verfahren zur Herstellung einer biologisch aktiven Erythropoietin-Isoform, wobei das Verfahren umfaßt, daß eine biologisch aktive Erythropoietin-Isoform mit einem einzigen isoelektrischen Punkt und mit einer spezifischen Anzahl von Sialinsäuren pro Erythropoietin-Molekül isoliert wird, wobei besagte Anzahl ausgewählt ist aus der Gruppe, die aus 1 bis 14 besteht.

2. Ein Verfahren nach Anspruch 1, wobei besagte Isoform durch die Expression einer exogenen DANN-Sequenz in einer nicht-menschlichen eukaryontischen Wirtszelle produziert wird.

3. Ein Verfahren nach Anspruch 1, wobei besagte Isoform Erythropoietin mit der Aminosäuresequenz von 1-165- oder 1-166-Human-Erythropoietin umfaßt.

4. Ein Verfahren nach Anspruch 1, wobei besagte Isoform 14 Sialinsäuren pro Erythropoietin-Molekül aufweist.

5. Ein Verfahren nach Anspruch 1, wobei besagte Isoform 13 Sialinsäuren pro Erythropoietin-Molekül aufweist.

6. Ein Verfahren nach Anspruch 1, wobei besagte Isoform 10 Sialinsäuren pro Erythropoietin-Molekül aufweist.

7. Ein Verfahren nach Anspruch 2, wobei besagte eukaryontische Wirtszelle eine CHO-Zelle ist.

8. Ein Verfahren zur Herstellung einer pharmazeutischen Zusammensetzung, wobei das Verfahren umfaßt, daß ein therapeutische wirksame Menge einer Erythropoietin-Isoform, hergestellt nach Anspruch 1, mit einem pharmazeutisch annehmbaren Verdünnungsmittel, Adjuvans oder Trägermittel kombiniert wird.

9. Ein Verfahren zur Herstellung von Erythropoietin, wobei das Verfahren im wesentlichen umfaßt, daß biologisch aktive Erythropoietin-Moleküle mit einer identischen Anzahl von Sialinsäuren pro Molekül hergestellt werden, wobei besagte Anzahl ausgewählt ist aus der Gruppe, die aus 1 bis 14 besteht.

10. Ein Verfahren nach Anspruch 9, in dem es 14 Sialinsäuren pro Erythropoietin-Molekül gibt.

11. Ein Verfahren nach Anspruch 9, in dem es 13 Sialinsäuren pro Erythropoietin-Molekül gibt.

12. Ein Verfahren nach Anspruch 9, in dem es 10 Sialinsäuren pro Erythropoietin-Molekül gibt.

13. Ein Verfahren nach Anspruch 9, wobei besagtes Molekül produziert wird durch die Expression einer exogenen DNA-Sequenz in einer nicht-menschlichen eukaryontischen Wirtszelle.

14. Ein Verfahren zur Herstellung einer pharmazeutischen Zusammensetzung, wobei das Verfahren umfaßt, daß eine therapeutische wirksame Menge Erythropoietin, hergestellt nach dem Verfahren von Anspruch 9, mit einem pharmazeutisch annehmbaren Verdünnungsmittel, Adjuvans oder Trägermittel kombiniert wird.

15. Ein Verfahren zur Herstellung einer Erythropoietin-Isofrom nach Anspruch 1, welches die Schritte umfaßt, daß ein gereinigtes Erythropoietin einer präparativen isoelektrischen Fokussierung unterzogen und eine einzelne Isoform aus dem Gel eluiert wird.

16. Ein Verfahren zur Herstellung einer Mischung von Erythropoietin-Isoformen mit einer vorbestimmten Anzahl von Sialinsäuren pro Molekül, wobei besagte Anzahl größer als 11 ist, welches umfaßt, daß Material, das Erythropoietin enthält, einer Ionenaustauschchromatographie unterzogen wird.

17. Ein Verfahren zur Herstellung einer Mischung von Erythropoietin-Isoformen mit einer vorbestimmten Anzahl von Sialinsäuren pro Molekül, wobei besagte Anzahl größr als 11 ist, welches umfaßt, daß ein Material, das Erythropoietin enthält, einer Chromatofokussierung unterzogen wird.

18. Ein Verfahren zum Erhalten einer Erythropoietin-Zusammensetzung mit einer vorbestimmten Anzahl von Sialinsäuren pro Molekül, welches umfaßt, daß eine Mischung aus zwei oder mehr Erythropoietin-Isoformen, die gemäß dem Verfahren von Anspruch 1 hergestellt sind, hergestellt wird, wobei besagte Mischung im wesentliche aus wenigstens zwei Isoformen mit weniger als 12 Sialinsäuren pro Molekül besteht oder wobei besagte Mischung im wesentlichen aus Erythropoietin-Isoforrnen mit 9, 10 und 11 Sialinsäuren pro Molekül besteht oder wobei besagte Mischung im wesentlichen aus wenigstens zwei Isoformen mit mehr als 11 Sialinsäuren pro Molekül besteht.

19. Das Verfahren nach Anspruch 18, wobei besagte Mischung im wesentlichen aus Erythropoietin-Isoformen mit 13 und 14 Sialinsäuren pro Molekül besteht.

20. Ein Verfahren nach Anspruch 18, zur Verwendung in einem Verfahren zur Erhöhung der Hämatokritgehalte in Säugetieren.

21. Ein Verfahren nach Anspruch 19, wobei besagtes Erythropoietin die Aminosäuresequenz von Human-Erythropoietin aufweist.

## Revendications (Revendications pour l'(les) Etat(s) contractant(s) suivant(s): AT, BE, CH, DE, DK, FR, GB, IT, LI, LU, VL, SE)

1. Isoforme d'érythropoïétine isolée biologiquement active, ayant un point isoélectrique unique et ayant un nombre spécifique d'acides sialiques par molécule d'érythropoïétine, ledit nombre étant choisi dans le groupe compris entre 1 à 14.

2. Isoforme d'érythropoïétine selon la revendication 1, dans laquelle ladite isoforme est le produit de l'expression d'une séquence d'ADN exogène dans une cellule hôte eucaryote non-humaine.

3. isoforme d'érythropoïétine selon la revendication 1, dans laquelle ladite isoforme comprend de l'érythropoïétine ayant la séquence d'acides aminés de l'érythropoïétine humaine 1-165 ou 1-166.

4. Isoforme d'érythropoïétine selon la revendication 1 ayant 14 acides sialiques par molécule d'érythropoïétine.

5. Isoforme d'érythropoïétine selon la revendication 1 ayant 13 acides sialiques par molécule d'érythropoïétine.

6. Isoforme d'érythropoïétine selon la revendication 1 ayant 10 acides sialiques par molécule d'érythropaïétine.

7. Isoforme d'érythropoïétine selon la revendication 2, dans laquelle ladite cellule hôte eucaryote est une cellule CHO,

8. Composition pharmaceutique comprenant une quantité thérapeutiquement efficace de ladite isoforme d'érythropoïétine de la revendication 1 et un support, adjuvant ou diluant pharmaceutiquement acceptable.

9. Composition constituée essentiellement de deux ou trois isoformes d'érythropoïétine selon la revendication 1.

10. Composition selon la revendication 9 constituée essentiellement d'isoformes d'érythropoïétine ayant moins de 12 acides sialiques par molécule.

11. Composition selon la revendication 10 constituée essentiellement d'isoformes d'érythropoïétine ayant 9, 10 et 11 acides sialiques par molécule d'érythropoïétine.

12. Composition selon la revendication 9 constituée essentiellement d'isoformes d'érythropoïétine ayant plus de 11 acides sialiques par molécule.

13. Composition selon la revendication 12 constituée essentiellement d'isoformes d'érythropoïétine ayant 13 et 14 acides sialiques par molécule.

14. Erythropoïétine constituée essentiellement de molécules d'érythropoïétine biologiquement actives ayant un nombre identique d'acides sialiques par molécule, ledit nombre étant choisi dans le groupe compris entre 1 à 14.

15. Erythropoïétine selon la revendication 14 ayant 14 acides sialiques par molécule d'érythropoïétine.

16. Erythropoïétine selon la revendication 14 ayant 13 acides sialiques par molécule d'érythropoïétine.

17. Erythropoïétine selon la revendication 14 ayant 10 acides sialiques par molécule d'érythropoïétine.

18. Erythropoïétine selon la revendication 14, dans laquelle ladite molécule est le produit de l'expression d'une séquence d'ADN exogène dans une cellule höte eucaryote non-humaine.

19. Erythropoïétine selon la revendication 13, dans laquelle ladite érythropoïétine présente la séquence d'acides aminés de l'érythropoïétine humaine.

20. Composition pharmaceutique comprenant une quantité thérapeutiquement efficace d'érythropoïétine de la revendication 14 et un support, adjuvant ou diluant pharmaceutiquement acceptable.

21. Procédé pour préparer une isoforme d'érythropoïétine selon la revendication 1, comprenant les étapes consistant à soumettre une érythropoïétine purifiée à une concentration isoélectrique préparative, et à éluer une isoforme unique à partir du gel.

22. Procédé de préparation d'un mélange d'isoformes d'érythropoïétine ayant un nombre prédéterminé d'acides sialiques par molécule, ledit nombre étant supérieur à 11, comprenant le fait de soumettre la matière contenant l'érythropoïétine à une chromatographie par échange d'ions.

23. Procédé de préparation d'un mélange d'isoformes d'érythropoïétine ayant un nombre prédéterminé d'acides sialiques par molécule, ledit nombre étant supérieur à 11, comprenant le fait de soumettre une matière contenant de l'érythropoïétine à une concentration chromatographique.

24. Composition selon la revendication 9 utilisable dans un procédé pour augmenter les taux d'hématocrite chez des mammifères.

25. Procédé pour obtenir une composition d'érythropoïétine ayant un nombre prédéterminé d'acides sialiques par molécule, comprenant la préparation d'un mélange de deux ou plus isoformes d'érythropoïétine selon la revendication 1,
dans lequel ledit mélange est constitué essentiellement d'au moins deux isoformes ayant moins de 12 acides sialiques par molécule, ou
dans lequel ledit mélange est constitué essentiellement d'isoformes d'érythropoïétine ayant 9, 10 et 11 acides sialiques par molécule, ou
dans lequel ledit mélange est constitué essentiellement d'au moins deux isoformes ayant plus de 11 acides sialiques par molécule, ou
dans lequel ledit mélange est constitué essentiellement d'isoformes d'érythropoïétine ayant 13 et 14 acides sialiques par molécule.

## Revendications (Revendications pour l'(les) Etat(s) contractant(s) suivant(s): GR, ES)

1. Procédé pour préparer une isoforme d'érythropoïétine biologiquement active, lequel procédé comprend l'isolation d'une isoforme d'érythropoïétine biologiquement active ayant un point isoélectrique unique et ayant un nombre spécifique d'acides sialiques par molécule d'érythropoïétine, ledit nombre étant choisi dans le groupe compris entre 1 à 14.

2. Procédé selon la revendication 1, dans lequel ladite isoforme est produite par l'expression d'une séquence d'ADN exogène dans une cellule hôte eucaryote non-humaine.

3. Procédé selon la revendication 1, dans lequel ladite isoforme comprend de l'érythropoïétine ayant la séquence d'acides aminés de l'érythropoïétine humaine 1-165 ou 1-166.

4. Procédé selon la revendication 1, dans lequel ladite isoforme présente 14 acides sialiques par molécule d'érythropoïétine.

5. Procédé selon la revendication 1, dans lequel ladite isoforme présente 13 acides sialiques par molécule d'érythropoïétine.

6. Procédé selon la revendication 1, dans lequel ladite isoforme présente 10 acides sialiques par molécule d'érythropoïétine.

7. Procédé selon la revendication 2, dans lequel ladite cellule hôte eucaryote est une cellule CHO.

8. Procédé de préparation d'une composition pharmaceutique, lequel procédé comprend la combinaison d'une quantité thérapeutiquement efficace d'une isoforme d'érythropoïétine préparée selon la revendication 1 avec un support, adjuvant ou diluant pharmaceutiquement acceptable.

9. Procédé de préparation d'érythropoïétine, lequel procédé comprend essentiellement la préparation de molécules d'érythropoïétine biologiquement actives ayant un nombre identique d'acides sialiques par molécule, ledit nombre étant choisi dans le groupe compris entre 1 à 14.

10. Procédé selon la revendication 9, dans lequel il y a 14 acides sialiques par molécule d'érythropoïétine.

11. Procédé selon la revendication 9, dans lequel il y a 13 acides sialiques par molécule d'érythropoïétine.

12. Procédé selon la revendication 9, dans lequel il y a 10 acides sialiques par molécule d'érythropoïétine.

13. Procédé selon la revendication 9, dans lequel ladite molécule est produite par l'expression d'une séquence d'ADN exogène dans une cellule hôte eucaryote non-humaine.

14. Procédé de préparation d'une composition pharmaceutique, lequel procédé comprend la combinaison d'une quantité thérapeutiquement efficace d'érythropoïétine préparée selon le procédé de la revendication 9 avec un support, adjuvant ou diluant pharmaceutiquement acceptable.

15. Procédé de préparation d'une isoforme d'érythropoïétine selon la revendication 1, comprenant les étapes consistant à soumettre une érythropoïétine purifiée à une concentration isoélectrique préparative, et à éluer une isoforme unique à partir du gel.

16. Procédé de préparation d'un mélange d'isoformes d'érythropoïétine ayant un nombre prédéterminé d'acides sialiques par molécule, ledit nombre étant supérieur à 11, comprenant le fait de soumettre la matière contenant l'érythropoïétine à une chromatographie par échange d'ions.

17. Procédé de préparation d'un mélange d'isoformes d'érythropoïétine ayant un nombre prédéterminé d'acides sialiques par molécule, ledit nombre étant supérieur à 11, comprenant le fait de soumettre une matière contenant de l'érythropoïétine à une concentration chromatographique.

18. Procédé pour obtenir une composition d'érythropoïétine ayant un nombre prédéterminé d'acides sialiques par molécule, comprenant la préparation d'un mélange de deux ou plusieurs isoformes d'érythropoïétine préparées selon le procédé de la revendication 1 ,
dans lequel ledit mélange est constitué essentiellement d'au moins deux isoformes ayant moins de 12 acides sialiques par molécule, ou
dans lequel ledit mélange est constitué essentiellement d'isoformes d'érythropoïétine ayant 9, 10 et 11 acides sialiques par molécule, ou
dans lequel ledit mélange est constitué essentiellement d'au moins deux isoformes ayant plus de 11 acides sialiques par molécule.

19. Procédé selon la revendication 18, dans lequel ledit mélange est constitué essentiellement d'isoformes d'érythropoïétine ayant 13 et 14 acides sialiques par molécule.

20. Procédé selon la revendication 18, utilisable dans un procédé pour augmenter les taux d'hématocrite chez des mammifères.

21. Procédé selon la revendication 19, dans lequel ladite érythropoïétine présente la séquence d'acides aminés de l'érythropoïétine humaine.
